# EUROPEAN PATENT SPECIFICATION

(11) **EP 4 326 524 B1**
(45) Date of publication and mention of the grant of the patent: **22.07.2026**
(21) Application number: 22792717.5
(22) Date of filing: 22.04.2022
(51) Int. Cl.: A61M 16/04

(54) **ENDOTRACHEAL TUBE SUPPORT DEVICES**
STÜTZVORRICHTUNGEN FÜR ENDOTRACHEALTUBUS
DISPOSITIFS DE SUPPORT DE TUBE ENDOTRACHÉAL

(30) Priority: 23.04.2021 US 202163179130 P
(43) Date of publication of application: 28.02.2024
(73) Proprietor: The Regents of the University of California, Oakland, CA 94607-5200 (US)
(72) Inventor: VAHABZADEH-HAGH, Andrew, La Jolla, California 92037 (US)
(74) Representative: Müller-Boré & Partner Patentanwälte PartG mbB
(86) International application number: PCT/US2022/071882
(87) International publication number: WO 2022/226541

(56) References cited:
- WO-A1-2020/000032
- US-A- 5 398 679
- US-A1- 2006 276 694
- US-A1- 2009 264 867
- US-A1- 2010 154 800
- US-A1- 2010 256 451
- US-A1- 2010 312 059
- US-A1- 2011 196 204
- US-A1- 2012 059 223
- US-A1- 2014 128 820
- US-A1- 2018 236 195

## Description

### TECHNICAL FIELD

This patent document relates to endotracheal tubes and, in particular, devices for controlling the shape of endotracheal tubes to prevent patient injury.

### BACKGROUND

Endotracheal tubes are used to provide mechanical ventilation for patients undergoing surgical procedures that require general anesthesia and for patients with various forms of respiratory failure. One million U.S. residents require invasive mechanical ventilation per year not counting short term intubation. Some patients may be intubated for several days to weeks. The presence of an endotracheal tube in the airway / trachea is not without complications that can result in laryngeal scarring and stenosis after the patient is extubated. The laryngeal scarring can be very difficult to successfully treat. As such, new devices and techniques are needed to prevent patient injury from endotracheal tubes.

Document US 2010/256451 A1 describes s laryngoscope insertion section comprising a tube guide comprising at least an inferior tube guiding member and a superior tube guiding member. The thickness of the insertion section in a first region is less than the external diameter of the largest diameter endotracheal tube in an operating range of endotracheal tube sizes plus the thickness of the inferior tube guiding member plus the thickness of the superior tube guiding member

Document US 2018/236195 A1 describes systems and devices for monitoring, detecting, and removing fluid build-up found at various regions along a tracheal tube of an intubated patient. The fluid management system includes pressure and flow sensors for detecting whether there is fluid at the various regions along the tracheal tube, and a means for drawing out the fluid into collection jars. The system also includes lavage features that is able to rinse different the various regions along a tracheal tube. Also disclosed are respiration insertion devices that either couple to existing tracheal tubes or incorporate tracheal tubing, where the respiration insertion body has channels and ports that contact various regions along the tracheal tube.

Document US 2012/059223 A1 describes a laryngoscope insertion section which includes an elongate member and a tube guide having a moveable guiding member, located transversely of the elongate member and moveable relative to adjacent elongate member to adjust the path of a retained endotracheal tube.

Document WO 2020/000032 A1 describes an intubation device for use in an endotracheal intubation procedure. The intubation device includes a laryngoscope blade having a tip and a base, and a handle attached to the base. The intubation device further includes a channel for receiving an endotracheal tube, the channel extending along the blade and partially along the handle, and the channel including an outlet proximate to the tip for allowing a distal end of the endotracheal tube to be advanced from the outlet. The intubation device further includes a tube movement mechanism in the handle for moving the endotracheal tube through the channel to thereby advance the endotracheal tube, the tube movement mechanism being configured to allow the user to hold the intubation device and advance the endotracheal tube in an endotracheal intubation procedure using a single hand. The intubation device further includes an auxiliary conduit for allowing an auxiliary function to be provided using the intubation device.

Document US 2010/312059 A1 describes a laryngoscope comprising a handle and an insertion section which extends from the handle, wherein the insertion section comprises a tube guide for retaining and guiding an endotracheal tube during intubation. The handle comprises at least one external tube engaging formation, such as a tube guiding member or tube retaining member. There is also disclosed a laryngoscope having a handle and insertion section which adapted to detachably retain and guide an endotracheal tube along a lateral side of the insertion section and handle such that a retained endotracheal tube is continuously curved, and preferably under flexural tension, from the most proximal location where the handle contacts the retained endotracheal tube to the most distal location where the insertion section contacts the superior side of a retained endotracheal tube.

### SUMMARY

The claimed invention is defined by the subject-matter of the independent claims. Preferred embodiments are defined by the dependent claims.

Disclosed are devices for bending endotracheal tubes or other medical tubes to cause the tubes to bend to a predetermined angle to prevent injury to a patient. In one aspect, an endotracheal tube bending apparatus is disclosed. The apparatus includes a backbone portion comprising at least a partially curved surface creating an angle between a proximate end of the apparatus and a distal end of the apparatus, and a plurality of curved extension portions structured to capture the endotracheal tube secured at a first predetermined location in a trachea against the backbone portion.

The apparatus can include the following features in various combinations. Each of the plurality of curved extension portions comprises a series of ridges structured to grip the endotracheal tube in place against the backbone portion. The backbone portion and the plurality of curved extension portions are structured to bend the endotracheal tube by the angle which is between 60 degrees and 90 degrees. The backbone portion and the plurality of curved extensions are structured to bend the tubing by the angle which is less than 60 degrees or greater than 90 degrees. The apparatus further includes an opening between adjacent extension portions. The apparatus further includes a rounded tab structure proximate to a first end of the apparatus oriented longitudinally along the apparatus. The apparatus further includes an extended notch in the backbone portion, the extended notch structured to engage a pilot balloon tube of the endotracheal tube fixing a position of the apparatus relative to the endotracheal tube. The rounded tab structure has a rotational position extending to an edge of one of the plurality of curved extension portions when viewed in cross-section at the first end of the apparatus, or has a rotational position opposite the one of the plurality of curved extension portions when viewed in cross-section at the first end of the apparatus. The rounded tab structure has a rotational position between an edge of one of the plurality of curved extension portions and opposite the one of the plurality of curved extensions when viewed in cross-section at the first end of the apparatus. The rounded tab structure has a hole for a safety string to pass therethrough to enable removal of the apparatus from a patient.

In another aspect, another endotracheal tube bending apparatus is disclosed. the apparatus includes a first prong attached to a base at a first proximate end and having a nub at a first distal end. The apparatus further includes a second prong and a third prong each attached to the base at a second and third proximate ends and connected together via a bridge structure at a second and third distal ends, wherein a pocket in the bridge is configured to capture the nub, and wherein the apparatus is structured to bend an endotracheal tube captured by the first, second, and third prongs after the endotracheal tube is secured at a first predetermined location in a trachea.

The following and other disclosed features can be included in various combinations. The first, the second, and the third prongs are configured to bend the endotracheal tube by a predetermined angle due to the nub being captured by the pocket. The predetermined angle is between 60 degrees and 90 degrees. The predetermined angle is less than 60 degrees or greater than 90 degrees.

In another aspect another endotracheal tube bending apparatus is disclosed. The apparatus includes a ring portion, and a strap portion emanating from the ring portion, wherein the strap portion is structured to bend the endotracheal tube passing across the ring portion in a plane of the ring portion causing a bending of the tube by an angle, wherein the endotracheal tube is secured at a first predetermined location in a trachea, and wherein the strap portion includes teeth on a surface of the strap. The apparatus further includes a pawl located in a hole in the ring opposite the emanating structured to engage the teeth allowing the strap to tighten and preventing the strap from loosening, wherein the ring portion, the strap portion, and the pawl are structured to bend the tube by the angle.

The apparatus can include the following and other disclosed features. The angle is between 60 degrees and 90 degrees. The ring portion, the strap portion, and the pawl are structured to bend the tube by the angle, and wherein the angle less than 60 degrees or greater than 90 degrees.

The disclosed apparatuses can be produced from materials including an acrylic material, ABS plastic, polyethylene, silicone, resin, or other plastic or rubber material, metal, metal alloy, or a combination thereof.

In an example, a method of intubation is disclosed. The method includes attaching an endotracheal tube bending apparatus to an endotracheal tube secured at a first predetermined location in a trachea to cause the endotracheal tube to bend by a predetermined angle at a second predetermined location along the endotracheal tube, wherein the bent endotracheal tube reduces or eliminates pressure or force on the patient's larynx.

The following and other disclosed features can be included. The predetermined angle is between 60 degrees and 90 degrees. The predetermined angle is less than 60 degrees or greater than 90 degrees.

### BRIEF DESCRIPTION OF THE DRAWINGS

FIG. 1 shows an example of orotracheal intubation with an endotracheal tube having a typical curve in the tube that may injure a patient and another curve caused by the disclosed device that will not injure the patient;
FIG. 2 shows an example of a typical endotracheal tube including a cuff, pilot balloon, and pilot balloon tube;
FIG. 3A shows a diagram of a sleeve type embodiment;
FIG. 3B shows images of example sleeve type embodiments;
FIG. 3C shows images of example sleeve type embodiments with a notch on the sleeve to engage a pilot balloon tube and a tab for adjusting the position of the sleeve on an endotracheal tube;
FIG. 3D shows diagrams of example sleeve type embodiments with the tab for adjusting the position of the sleeve on an endotracheal tube in a different position than shown in FIG. 3C
FIG. 3E shows diagrams showing different amounts of material removed along a sleeve type embodiment;
FIG. 4A shows an example diagram of a three-prong type embodiment;
FIG. 4B shows images of an example three-prong type embodiment;
FIG. 5 shows a diagram of an example clamping type embodiment;
FIG. 6A shows an image illustrating a typical bend in an endotracheal tube;
FIG. 6B shows an image illustrating a bend in an endotracheal tube with sleeve type clip placed on the tube producing a 60-degree;
FIG. 6C shows an image illustrating a bend in an endotracheal tube with a sleeve type clip placed on the tube producing a 70-degree bend;
FIG. 6D shows an image illustrating a bend in an endotracheal tube with a sleeve type clip placed on the tube producing a 90-degree bend;
FIG. 7 shows cross-sectional views of an example sleeve type embodiment; and
FIG. 8 shows an example of a process.

### DETAILED DESCRIPTION

Endotracheal tubes provide mechanical ventilation for patients. Standard endotracheal tube can cause forces and pressure on the larynx ('voicebox') causing injuries that can lead to scar formation and stenosis after the patient is extubated as well as other injuries including to the trachea and esophagus. A laryngeal scar referred to as a posterior glottic stenosis (PGS) can be formed that is very difficult to treat. Such patients can be left in need of a tracheostomy tube and without the ability to speak. Other injuries can also occur.

Disclosed is a device that can be placed onto the sidewall of a standard endotracheal tube to create a bend in the tube at a predetermined one or more positions along the length of the tube. The bend in the tube can eliminate or drastically reduce the pressure applied by the tube on the posterior aspect of the 'voicebox' / airway and trachea. In some example embodiments, the device is a bracket or sleeve that fits over the body of an endotracheal tube. The sleeve can be held in place on the tube by extensions that wrap partially or fully around the tube. The sleeve has sufficient rigidity to hold one portion of the tube at an angle with respect to another portion of the tube along the longitudinal axis of the tube without compromising luminal patency.

In other embodiments, the device has three "prongs" that a standard endotracheal tube passes through where the prongs cause the tube to bend with a predetermined angle. In some embodiments, a device can be integrated into the sidewall of the endotracheal tube itself to create a bend in the tube at the desired position along its length of the tube.

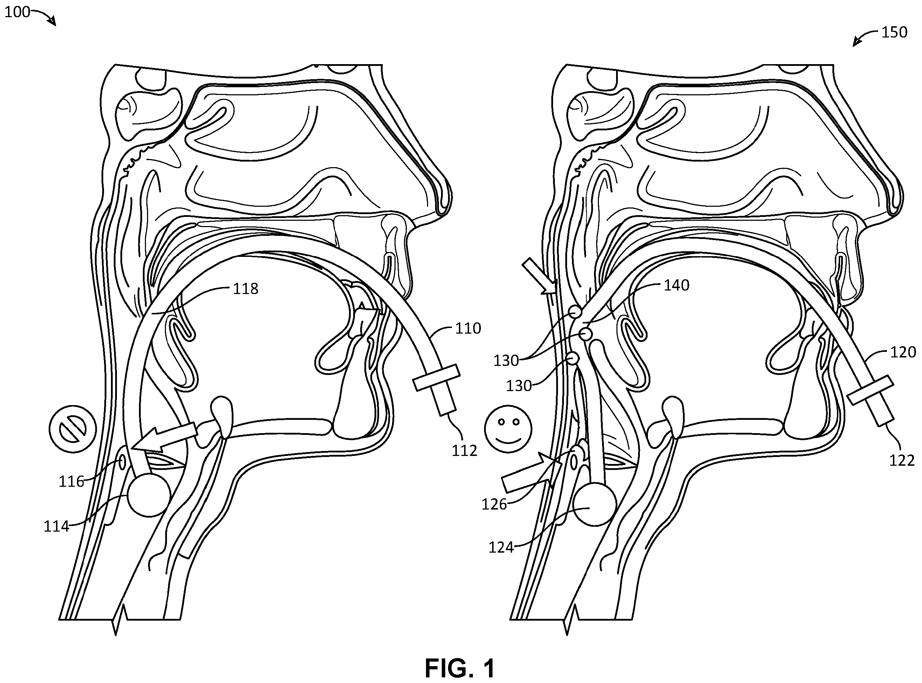

FIG. 1 at 100 shows an example of orotracheal intubation with an endotracheal tube 110 that has a curve 118 that would be typical after insertion using current medical techniques. First end 112 of endotracheal tube 110 connects to a ventilator and induces breathing. Second end 114 includes a cuff. The curve 118 is influenced by the stiffness and memory of the tubing and the forces on the tube from contact in the throat or trachea that cause the tube to bend. The curvature 118 of the tubing applies pressure on the posterior aspect of the larynx at 116. This pressure can cause injury to the patient. The tubing has enough stiffness to allow the tube to be inserted into the throat or trachea through the patient's mouth.

FIG. 1 at 150 shows an example of orotracheal intubation with an endotracheal tube 120 that has a curvature 140 that has been altered from the curvature 118 by one the devices disclosed in this patent document. In the example of FIG. 1 at 150, a device with three-prongs 130 is shown that changes the curvature of the endotracheal tube 120 such that the tube does not put pressure, or greatly reduces the pressure on the posterior aspect of the larynx at 126. Endotracheal tube 120 has a first end 122 that connects to a ventilator and a second end 124 that are similar, or the same, as the first and second ends shown at 100. In the example at 150, the curvature 140 is influenced by the stiffness of the tubing and the forces on the tube from the three prongs 130 of the disclosed device in contact with the tube. The curvature 140 of the tubing applies no pressure, or nearly no pressure, on the posterior aspect of the larynx at 126. This lack of pressure prevents injury to the patient.

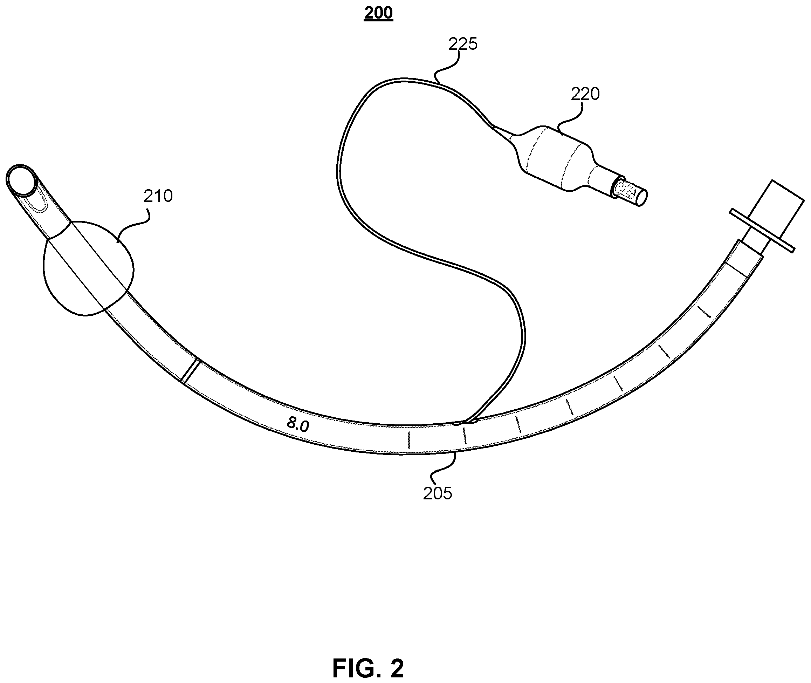

FIG. 2 shows an example of a typical endotracheal tube 200 including tube 205, cuff 210, pilot balloon 220, and pilot balloon tube 225. The endotracheal tube has a cuff 210 near the end of the tube at the end of the tube 205 that is inserted into a patient. The cuff 210 expands to provide a seal ensuring that air or oxygen from the ventilator is injected into the patient's lungs. Pilot balloon 220 is used to indicate whether the cuff 210 is inflated or not. When the cuff 210 is inflated, then pilot balloon 220 is inflated, and when cuff 210 is not inflated, then pilot balloon 220 is not inflated. Pilot balloon 220 is inflated via pilot balloon tube 225 through which air passes between the cuff to the pilot balloon. In some example embodiments of the disclosed device, an engagement mechanism incorporated into the device may be used to engage the pilot balloon tube to stop sliding movement of the device along the tube 205 toward the cuff 210. By stopping further sliding, the device is fixed at a known location along tube 205 and the device is prevented from moving farther than necessary toward the cuff.

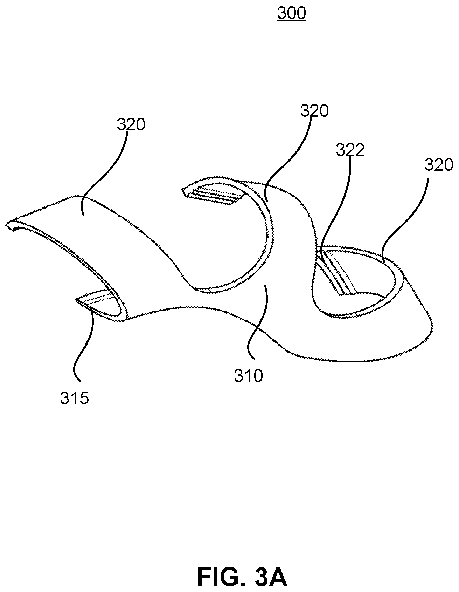

FIG. 3A at 300 shows an example of a sleeve type embodiment. In the example shown at 300, the endotracheal tube (not shown) is pressed into sleeve 310. The sleeve has curved extensions 320 that wrap around the endotracheal tube. As viewed from the end of sleeve 310, the curved extensions 320 can wrap around so that sleeve 310 and curved extensions 320 are in contact with more than 180 degrees around the tube. For example, the extensions may extend to cover 190 or 200 degrees of the tube, or another angle. Covering more than 180 degrees of the tubing aids in capturing the tubing in the sleeve. In some embodiments, ridges 322 on the inside portion of the sleeve may be used to further grip and hold the tubing in position inside the sleeve. FIG. 3A at 300 shows a sleeve with three extensions and two openings in the sleeve. Some embodiments may have more or fewer extensions and openings. For example, an embodiment may have four extensions and three openings. In some example embodiments, the sleeve may have no openings or extensions. In these embodiments, the tube may be inserted through the sleeve rather than having extensions that clip to the tube. Various embodiments of the sleeve 310 can cause the endotracheal tubing to bend 60-90 degrees, or another angle. In some example embodiments, sleeve 300 is described as having a backbone 315 which together with curved extensions 320 hold a captured endotracheal tube in a bent position. The bent endotracheal tube prevents pressure from being placed on the posterior aspect of the larynx thereby preventing injury to the patient.

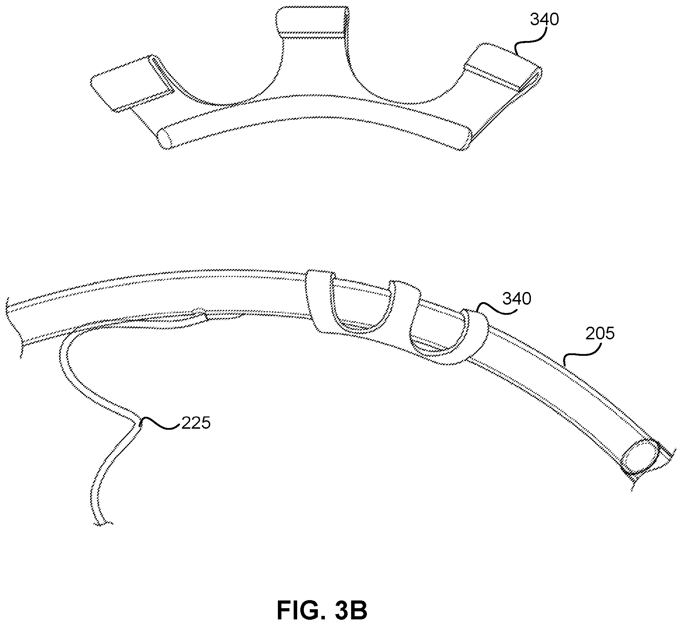

FIG. 3B show images of an example of a sleeve type embodiment. FIG. 3B shows the sleeve at 340 and the tube at 205. Pilot balloon tube 225 is also shown in the image.

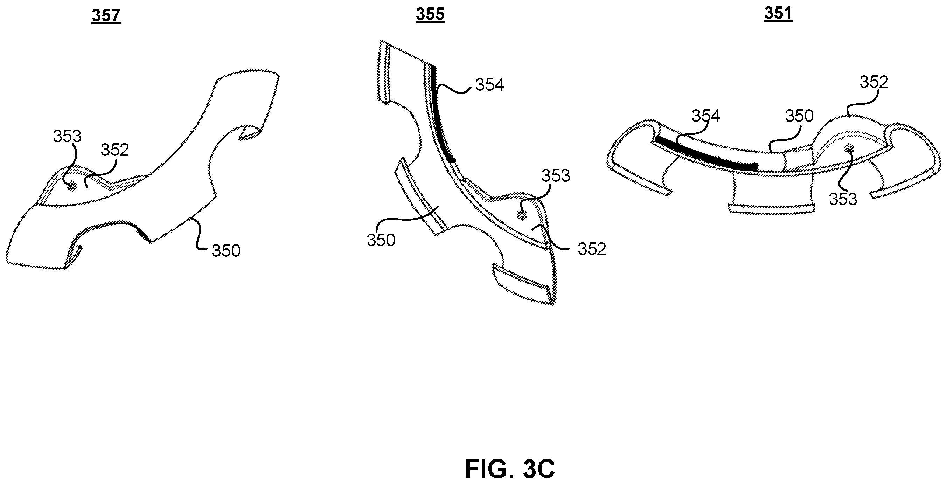

FIG. 3C shows images of an example sleeve type embodiment 350 with an notch or engagement slot 354 to engage a pilot balloon tube 225 and a tab 352 to allow for the application of pressure to the sleeve to move the sleeve along the endotracheal tube or rotate the sleeve about the tube. The notch or engagement slot 354 is an area of sleeve 350 where the sleeve material is removed in order to create a notch or slot. As the sleeve 350 is moved down an endotracheal tube, the notch or engagement slot 352 captures the pilot balloon tube 225 exiting the endotracheal tube and stops further sliding of the sleeve 350 down the endotracheal tube. The notch or engagement slot 354 allows for consistent positioning of the sleeve with respect to pilot balloon tubing that exits from the side of the endotracheal tube. Shown at 357 is an outer portion of an example sleeve and the tab 352 with a hole 353 in the tab. Shown at 355 is the inner aspect of the example sleeve. Shown at 351 is the inner and superior aspect of the example sleeve.

The sleeve type embodiment shown in FIG. 3C is similar to the embodiments described above with the addition of tab 352 to allow adjustment of the sleeve along the endotracheal tube, the hole 352 to allow for a safety string, and a notch or engagement slot 354. The tab 352 is located on the superior aspect of the sleeve to allow easy adjustment to the sleeve position once anchored onto the endotracheal tube. The tab 352 can be located in a rotational aspect anywhere there is material for the tab; the tab is not positioned in an opening of the sleeve. In some example embodiments, the tab is placed in a rotational position that is opposite a location mid-way through an opening as shown at 352. In this position, the tab is along the inner portion of the sleeve. Hole or eyelet 353 (shown with an asterisk) is placed through the tab 352 to facilitate attaching a safety string (not shown) to the sleeve. The safety string allows for removal of the sleeve from a patient should the sleeve become dislodged from the endotracheal tube in the mouth and needing to be retrieved. The safety string can be a loop that passes through the hole or eyelet or a length of string with a stopper or knot to prevent the string from being pulled through the hole or eyelet 353.

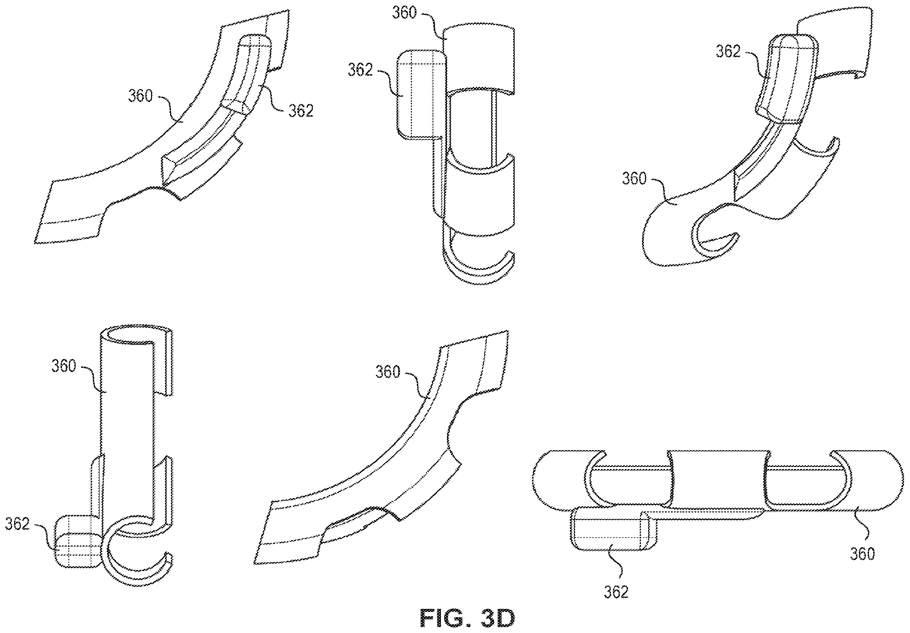

In another example embodiment shown in FIG. 3D, sleeve 360 has a tab 362 that is placed in a rotational position that is opposite a location of an opening in the sleeve or rotated about 90 degrees clockwise (when viewed from the end of the sleeve closest to the tab) from the location of tab 352 shown in FIG. 3C. In this position, the tab is along the inner portion of the sleeve. A notch, hole or eyelet, and safety string (not shown) can be included in sleeve shown in FIG. 3D similar to those features described with respect to FIG. 3C. Alternatively, the tab can also be placed at a rotational position between that shown in FIGs. 3C and 3D, or even in another rotational position on the sleeve or translated down the sleeve.

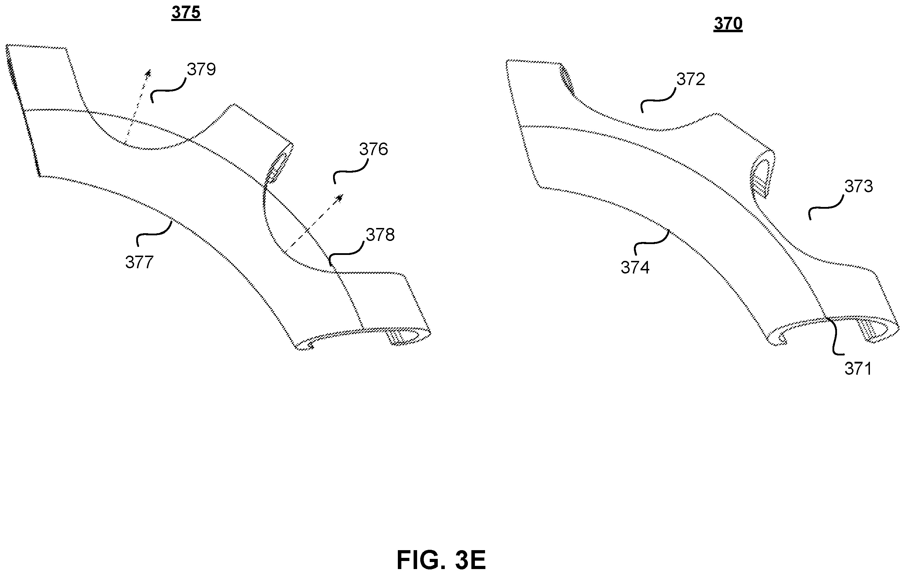

FIG. 3E shows diagrams with different amounts of material removed corresponding to the openings in a sleeve type embodiment. At 370, sleeve 374 has material removed at openings 372 and 373. A reference line 371 is shown indicating the center of the sleeve along its length. The material removed at 372 and 373 can be the same where openings 372 and 373 having the same shape, or openings 372 and 373 can have different shapes (not shown) with different amounts of material removed. At 375, sleeve 377 is similar to sleeve 374 and has openings 379 and 376 that can be the same or different shapes as described above. A reference line 378 is also shown. As shown in the example of FIG. 3E, more material is removed corresponding to larger openings at 376 and 379 than at 372 and 373.

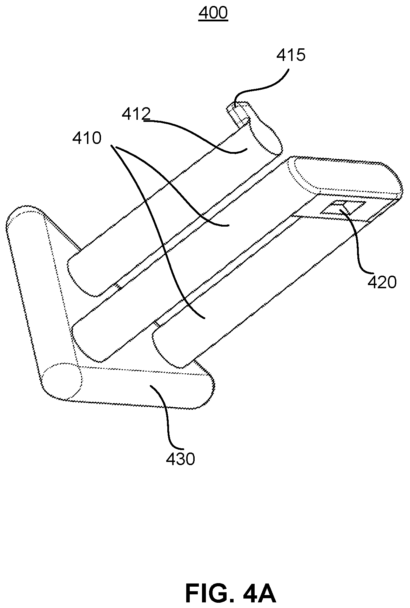

FIG. 4A at 400 shows an example of a three-prong type embodiment. In the example shown at 400, the endotracheal tube (not shown) is pressed or slid between prong 412 and prongs 410. In the diagram at 400, the endotracheal tube passes from out of the page on the left, between prong 412 and prongs 410, to past the page on the right. After the tube is passed between the prongs, prong 412 may be bent toward prongs 410 and prongs 410 bent toward prong 412, then prong 412 is bent past the prongs 410 such that nub 415 engages into pocket 420 thereby holding prong 412 in position. The bending of prongs 410 and 412 causes the endotracheal tube to bend in a plane parallel to base 430. Various embodiments of the three-prong device can cause the endotracheal tubing to bend 60-90 degrees, or another angle. The bending of the endotracheal tube prevents pressure from being placed on the posterior aspect of the larynx thereby preventing injury to the patient

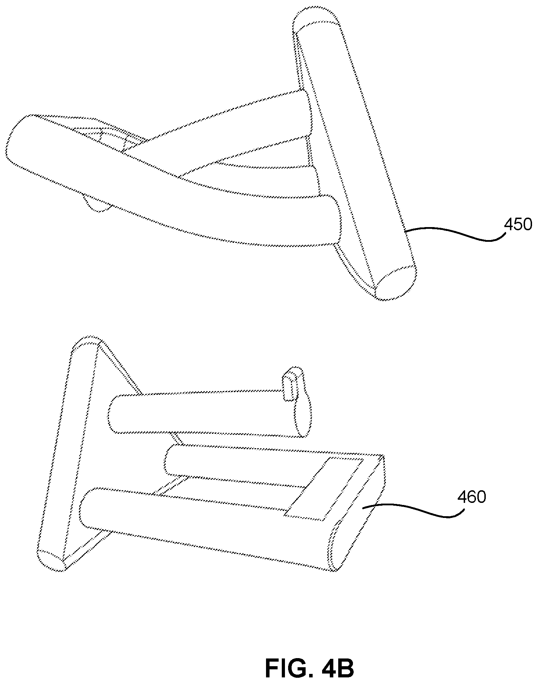

FIG. 4B shows images of an example of a three-prong type embodiment. Shown at 450 is a three-prong device before the prongs are bent toward one another and the nub engaged with the pocket. Shown at 450 is the same three-prong after the prongs are bent toward one another and the nub is engaged with the pocket. No endotracheal tube is shown at 450 but if the tubing was present would pass through the page. At 460, the tube, if present, would pass from out of the page to the left to past the page to the right.

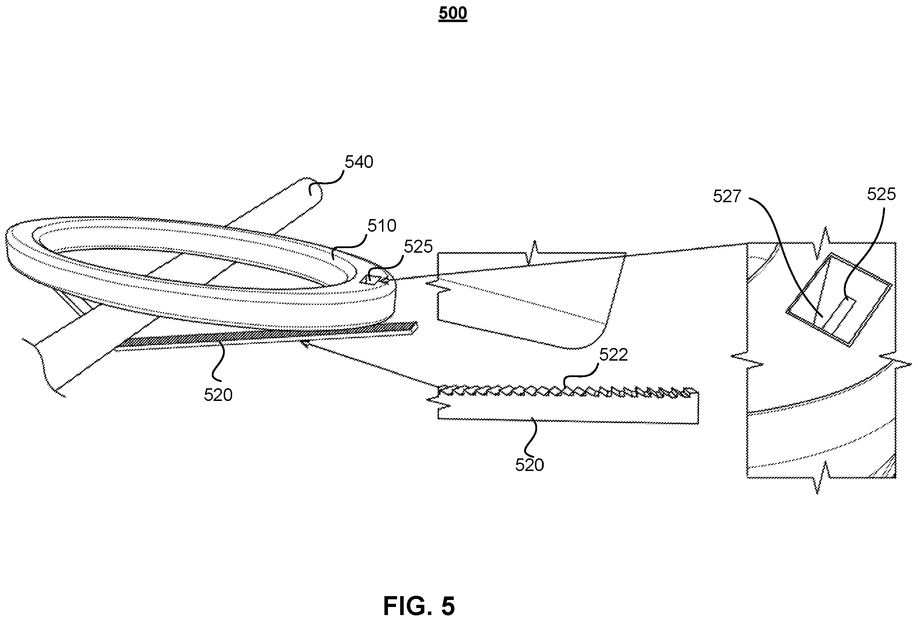

FIG. 5 depicts example diagrams 500 of a clamping type embodiment. The clamping type embodiment includes ring 510 and strap 520 that includes teeth 522 that engage a pawl 527 in an opening 525 of the ring. The portion of the strap 520 with teeth 522 pulled in a forward direction through the pawl 527 is locked by the pawl 527 and will not slide in a reverse direction. In some example embodiments, the pawl can be released with a tool designed for that purpose. An endotracheal tube 540 is placed parallel to the plane of the ring 510. The strap 520 is placed over the tube 540 in position to provide pressure on the endotracheal tube 540 at a contact point along the strap 520 and two sides of the ring 510 to cause bending of the tube 540. The strap 520 is produced as part of the ring 510 or the ring and strap can be produced separately and attached together via thermal techniques. The amount of bending caused to the tube 540 can be controlled by how much length of the strap 520 is pulled through the pawl 527.

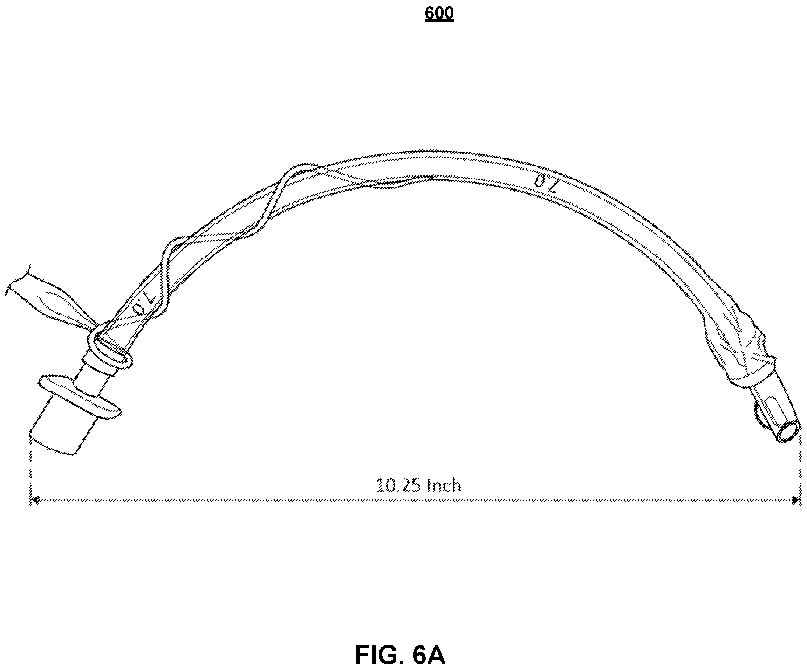

FIG. 6A shows an image 600 illustrating a typical bend in an endotracheal tube. In the image one end of the endotracheal tube is placed at a starting location, the tube is bent in a natural curve without placing pressure along the tube to adjust the curvature. In the example of FIG. 6A, the distance between the starting and ending locations is about 10.25 inches.

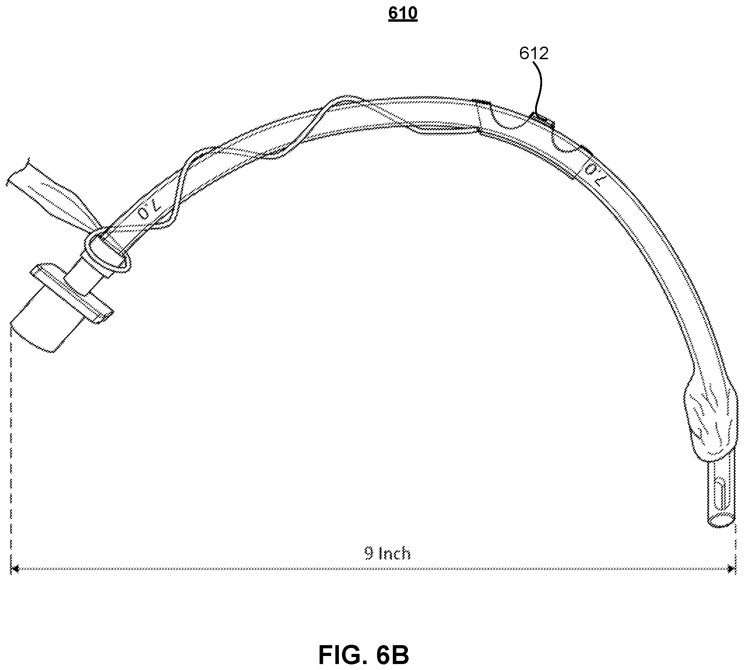

FIG. 6B shows an image 610 illustrating a bend in an endotracheal tube with sleeve type embodiment 612 placed on the tube producing a 60-degree bend. In the image one end of the endotracheal tube is placed at a starting location, the tube is bent with the sleeve type embodiment 612 placed on the tube to adjust the curvature. In the example of FIG. 6B, the distance between the starting and ending locations is about 9 inches which is about 1.25 inches shorter than the example of FIG. 6A.

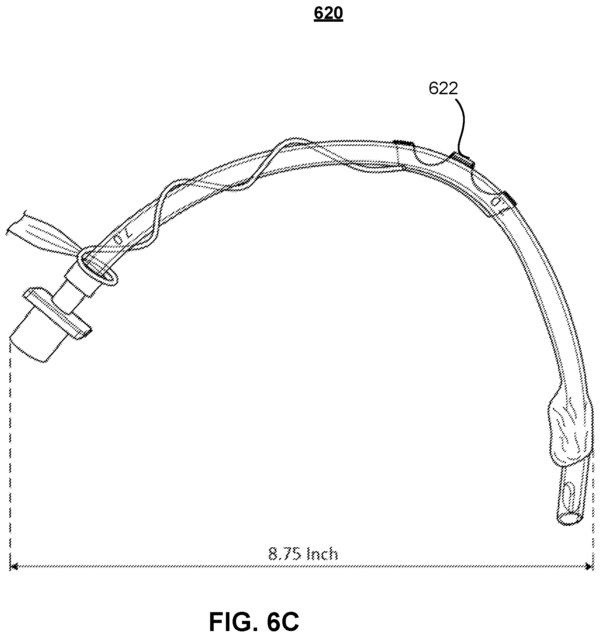

FIG. 6C shows an image 620 illustrating a bend in an endotracheal tube with sleeve type embodiment 622 placed on the tube producing a 70-degree bend. In the image one end of the endotracheal tube is placed at a starting location, the tube is bent with sleeve type embodiment placed on the tube to adjust the curvature. In the example of FIG. 6C, the distance between the starting and ending locations is about 8.75 inches which is about 1.50 inches shorter than the example of FIG. 6A.

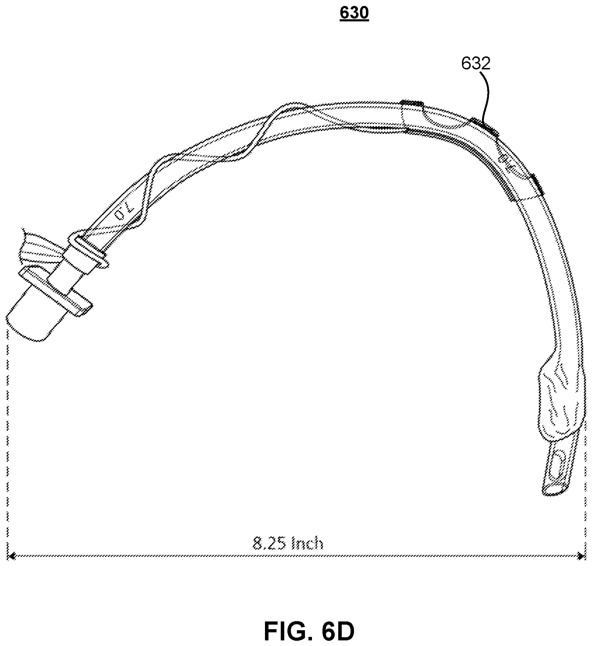

FIG. 6D shows an image 630 illustrating a bend in an endotracheal tube with sleeve type embodiment 632 placed on the tube producing a 90-degree bend. In the image one end of the endotracheal tube is placed at a starting location, the tube is bent with sleeve type embodiment placed on the tube to adjust the curvature. In the example of FIG. 6D, the distance between the starting and ending locations is about 8.25 inches which is about 2 inches shorter than the example of FIG. 6A. In the images shown in FIGs. 6B-6D, the sleeve was placed about 2/3 of the way along the tube from the starting location (ventilator end of the endotracheal tube) to the endling location (patient end of the endotracheal tube). The sleeve can be placed at other locations along the tube such as at the midpoint of the tube, or closer to the patient end (greater than 2/3 of the way from the starting location to the ending location).

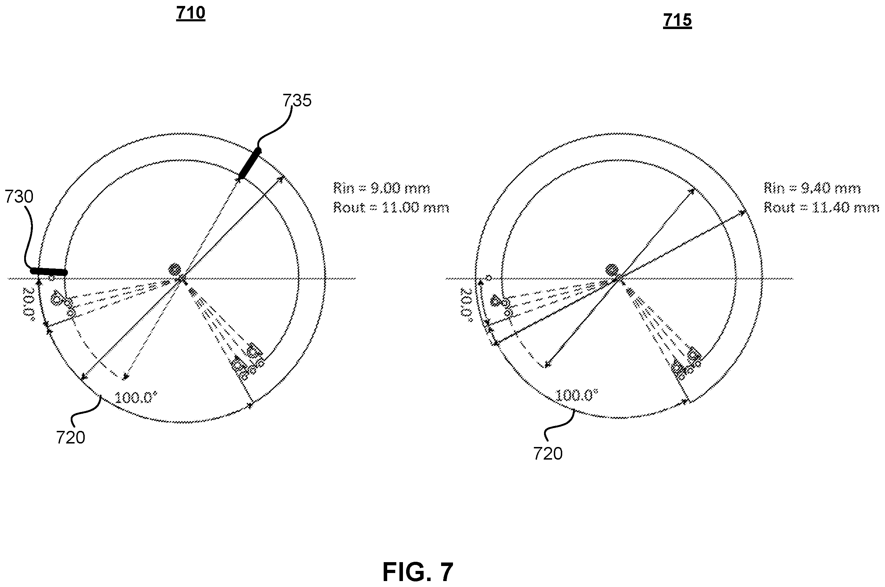

FIG. 7 shows cross-sectional views of an example sleeve type embodiment. In the embodiment shown at 710, the sleeve has an inner diameter of 9.00 mm and an outer diameter of 11.00 mm, and accordingly a nominal thickness of 2.00 mm. In the embodiment shown at 715, the sleeve has an inner diameter of 9.40 mm and an outer diameter of 11.40 mm, and a nominal thickness of 2.00 mm. The inside and outside diameters of the sleeve can be adjusted for different diameter endotracheal tubes with outer diameters ranging from about 3.4 mm to about 13 mm. The different endotracheal tube outer diameters are used on different sized patients from neonates (3.4 mm) to adults (up to 13 mm). Different sleeves with different inner and outer diameters may be used for different outer diameter endotracheal tubes. Similarly different sized three prong type embodiments and clamping type embodiments may be used for different sized endotracheal tubes and/or patient sizes/ages. The nominal thickness of 2 mm may also vary with smaller diameter endotracheal tubes having sleeves with nominal thicknesses much smaller than 2 mm. In an example embodiment, a sleeve with an inner diameter of 9 mm can be clipped to a endotracheal tube with outer diameter of 9.5 mm. In some embodiments, the sleeve inner diameter is slightly smaller in diameter than the endotracheal tube (0.1 - 0.75 mm) but in some embodiments the sleeve can have the same inner diameter as the outer diameter of the tube or even slightly larger. Each particular sleeve can be clipped to one or more endotracheal tube diameters. An cross-sectional view of the areas where the sleeve material is removed is shown at 720. In some embodiments the opening is about 100 degrees in arc but can vary in different embodiments from about 80 degrees to about 120 degrees.

Shown at 730 in FIG. 7 is the approximate rotational location of the tab 352 shown in FIG. 3C when the sleeve is viewed from the end closest to the tab 352. Shown at 735 in FIG. 7 is an alternate approximate radial location of the tab 362 shown in FIG. 3D when the sleeve is viewed from the end closest to the tab 352

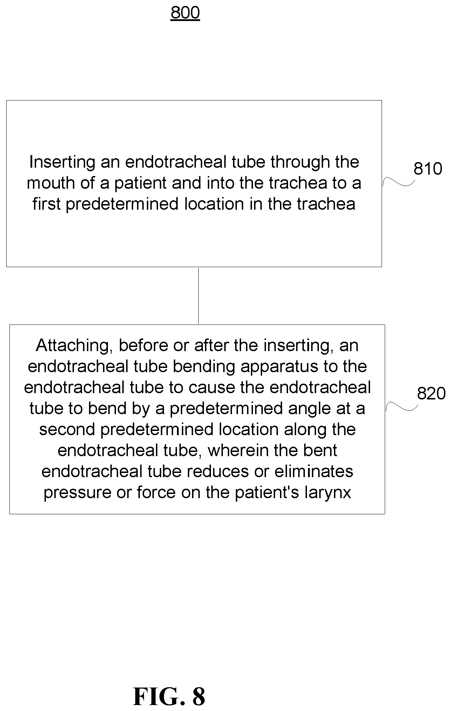

FIG. 8 depicts an example of a process. At 810, the process includes inserting an endotracheal tube through the mouth of a patient and into the trachea to a first predetermined location in the trachea. At 820, the process includes attaching, before or after the inserting, an endotracheal tube bending apparatus to the endotracheal tube to cause the endotracheal tube to bend by a predetermined angle at a second predetermined location along the endotracheal tube, wherein the bent endotracheal tube reduces or eliminates pressure or force on the patient's larynx. In some example embodiments, the endotracheal tube bending apparatus is applied to an endotracheal tube that is already positioned and secured in the airway. In this way, the endotracheal tube bending apparatus will not interfere with intubation or placement of the endotracheal tube through the mouth and into the airway / trachea.

The various embodiments may include one or more materials including one or more of acrylic, acrylonitrile butadiene styrene (ABS), nylon, nylon PA-12, polycarbonate, polyetheretherketone, polyethersulfone, polyethylene, polypropylene, polysulfone, polytetrafluoroethylene, polyurethane, polyvinylchloride, polyetherimide, silicone, resin (e.g., BioMed Clear Resin comprising 4,4'-isopropylidenediphenol, ethoxylated and 2-methylprop-2-enoic acid, urethane dimethacrylate, methacrylate monomer(s), photoinitiator(s)), or other plastic, rubber, or elastomeric materials. Some embodiments may be made from a single material and other embodiments may be made from a combination of materials. As an illustrative example, the base 280 in the three-prong embodiment may be made from a rigid material and the prongs from a more flexible material. Some embodiments may also include metals and metal alloys. In some example embodiments, a sleeve embodiment may include a backbone portion and a plurality of curved extension portions made in a single structure. The backbone portion maintains the curvature of an endotracheal tube captured by the plurality of curved portions. In another example, a sleeve embodiment may include a metal backbone to maintain the curvature of the tubing with a more flexible material being used for the sleeve. Desirable materials include biocompatible materials that are strong enough to hold a shape but are not too brittle. Suitable materials should be stable at body temperature, have high tensile strength, and low flexural modulus. Suitable materials can be flexible, semi-flexible, or rigid. In some example embodiments, the disclosed devices are 3-D printed.

Although a few variations have been described in detail above, other modifications or additions are possible. In particular, further features and/or variations may be provided in addition to those set forth herein. Moreover, the example embodiments described above may be directed to various combinations and subcombinations of the disclosed features and/or combinations and subcombinations of several further features disclosed above. In addition, the logic flow depicted in the accompanying figures and/or described herein does not require the particular order shown, or sequential order, to achieve desirable results. Other embodiments may be within the scope of the following claims.

Similarly, while elements are depicted in the drawings in a particular order, this should not be understood as requiring that the elements be assembled or performed in the particular order shown or in sequential order, or that all illustrated operations be performed, to achieve desirable results. Moreover, the separation of various elements in the embodiments described in this patent document should not be understood as requiring such separation in all embodiments.

Only a few implementations and examples are described and other implementations, enhancements and variations can be made based on what is described and illustrated in this patent document.

## Claims

1. An endotracheal tube bending apparatus (300), comprising a backbone portion (315) comprising at least a partially curved surface creating an angle between a proximate end of the apparatus and a distal end of the apparatus, wherein the endotracheal tube bending apparatus further comprises:
a plurality of curved extension portions (320) structured to capture an endotracheal tube (110, 120, 200) secured at a first predetermined location in a trachea against the backbone portion.

2. The endotracheal tube bending apparatus of claim 1, wherein each of the plurality of curved extension portions comprises a series of ridges structured to grip the endotracheal tube (110, 120, 200) in place against the backbone portion.

3. The endotracheal tube bending apparatus of claim 1 or claim 2, wherein the backbone portion and the plurality of curved extension portions are structured to bend the endotracheal tube (110, 120, 200) by the angle.

4. The endotracheal tube bending apparatus of any one of claims 1 to 3, further comprising:
a rounded tab structure proximate to a first end (112, 122) of the apparatus oriented longitudinally along the apparatus.

5. The endotracheal tube bending apparatus of claim 4, wherein the rounded tab structure has a rotational position extending to an edge of one of the plurality of curved extension portions when viewed in cross-section at the first end (112, 122) of the apparatus, or has a rotational position opposite the one of the plurality of curved extension portions when viewed in cross-section at the first end (112, 122) of the apparatus.

6. The endotracheal tube bending apparatus of claim 4, wherein the rounded tab structure has a rotational position between an edge of one of the plurality of curved extension portions and opposite the one of the plurality of curved extensions (320) when viewed in cross-section at the first end (112, 122) of the apparatus.

7. The endotracheal tube bending apparatus of any one of claims 4 to 6, wherein the rounded tab structure has a hole for a safety string to pass therethrough to enable removal of the apparatus from a patient.

8. The endotracheal tube bending apparatus of any one of claims 1 to 7, further comprising:
an extended notch in the backbone portion, the extended notch structured to engage a pilot balloon tube (225) of the endotracheal tube (110, 120, 200) fixing a position of the apparatus relative to the endotracheal tube (110, 120, 200).

9. An endotracheal tube bending apparatus (400), comprising:
a first prong (412) attached to a base at a first proximate end and having a nub (415) at a first distal end; and
a second prong (410) and a third prong (410) each attached to the base at a second and third proximate ends and connected together via a bridge structure at a second and third distal ends, wherein a pocket (420) in the bridge is configured to capture the nub (415), and wherein the apparatus is structured to bend an endotracheal tube (110, 120, 200, 540) captured by the first, second, and third prongs (412, 410) after the endotracheal tube (110, 120, 200, 540) is secured_at a first predetermined location in a trachea.

10. The endotracheal tube bending apparatus of claim 9, wherein the first, the second, and the third prongs (412, 410) are configured to bend the endotracheal tube (110, 120, 200, 540) by an angle due to the nub (415) being captured by the pocket (420).

11. An endotracheal tube bending apparatus (500), comprising:
a ring portion (510);
a strap portion (520) emanating from the ring portion, wherein the strap portion is structured to bend an endotracheal tube (110, 120, 200, 540) passing across the ring portion in a plane of the ring portion causing a bending of the tube by an angle, wherein the endotracheal tube (110, 120, 200, 540) is secured at a first predetermined location in a trachea, and wherein the strap portion includes teeth (522) on a surface of the strap; and
a pawl (527) located in a hole in the ring opposite the emanating structured to engage the teeth (522) allowing the strap to tighten and preventing the strap from loosening,
wherein the ring portion, the strap portion, and the pawl (527) are structured to bend the tube by the angle.

12. The apparatus of any one of claims 1 to 11, wherein the apparatus comprises an acrylic material, ABS plastic, polyethylene, silicone, resin, or other plastic or rubber material, metal, a metal alloy, or a combination thereof.

13. The endotracheal tube bending apparatus of any one of claims 1 to 12, wherein the angle is between 60 degrees and 90 degrees.

14. The endotracheal tube bending apparatus of any one of claims 1 to 12, wherein the angle is less than 60 degrees or greater than 90 degrees.

## Patentansprüche

1. Eine Endotrachealtubus-Biegevorrichtung (300), umfassend einen rückgratartigen Abschnitt (315), der eine zumindest teilweise gekrümmte Oberfläche umfasst, welche einen Winkel zwischen einem proximalen Ende der Vorrichtung und einem distalen Ende der Vorrichtung bildet, wobei die Endotrachealtubus-Biegevorrichtung ferner Folgendes umfasst:
eine Vielzahl von gekrümmten Erweiterungsabschnitten (320), die dazu ausgebildet sind, einen Endotrachealtubus (110, 120, 200), der an einer ersten vorbestimmten Position in einer Trachea gesichert ist, gegen den rückgratartigen Abschnitt zu sichern.

2. Die Endotrachealtubus-Biegevorrichtung nach Anspruch 1, wobei jeder der Vielzahl von gekrümmten Erweiterungsabschnitten eine Folge von Rippen umfasst, die dazu strukturiert sind, den Endotrachealtubus (110, 120, 200) an seinem Platz gegen den rückgratartigen Abschnitt zu sichern.

3. Die Endotrachealtubus-Biegevorrichtung nach Anspruch 1 oder Anspruch 2, wobei der rückgratartige Abschnitt und die Vielzahl von gekrümmten Erweiterungsabschnitten dazu ausgebildet sind, den Endotrachealtubus (110, 120, 200) um den Winkel zu biegen.

4. Die Endotrachealtubus-Biegevorrichtung nach irgendeinem der Ansprüche von 1 bis 3, ferner umfassend:
eine abgerundete Laschenstruktur in der Nähe eines ersten Endes (112, 122) der Vorrichtung, die in Längsrichtung entlang der Vorrichtung ausgerichtet ist.

5. Die Endotrachealtubus-Biegevorrichtung nach Anspruch 4, wobei die abgerundete Laschenstruktur eine Drehstellung aufweist, die sich zu einer Kante eines aus der Vielzahl von gekrümmten Erweiterungsabschnitten erstreckt, wenn man im Querschnitt am ersten Ende (112, 122) der Vorrichtung betrachtet, oder eine Drehstellung aufweist, die dem einen aus der Vielzahl von gekrümmten Erweiterungsabschnitten gegenüberliegt, wenn man im Querschnitt am ersten Ende (112, 122) der Vorrichtung betrachtet.

6. Die Endotrachealtubus-Biegevorrichtung nach Anspruch 4, wobei die abgerundete Laschenstruktur eine Drehstellung zwischen einer Kante eines aus der Vielzahl von gekrümmten Erweiterungsabschnitten und gegenüber dem einem aus der Vielzahl von gekrümmten Erweiterungsabschnitten (320) aufweist, wenn im Querschnitt am ersten Ende (112, 122) der Vorrichtung betrachtet.

7. Die Endotrachealtubus-Biegevorrichtung nach irgendeinem der Ansprüche von 4 bis 6, wobei die abgerundete Laschenstruktur ein Loch aufweist, durch das eine Sicherheitsschnur hindurchgeführt werden kann, um das Entfernen der Vorrichtung aus einem Patienten zu ermöglichen.

8. Die Endotrachealtubus-Biegevorrichtung nach irgendeinem der Ansprüche von 1 bis 7, ferner umfassend:
eine verlängerte Ausnehmung im rückgratartigen Abschnitt, wobei die verlängerte Ausnehmung dazu ausgebildet ist, mit einem Pilotballonschlauch (225) des Endotrachealtubus (110, 120, 200) in Eingriff zu kommen und dadurch eine Position der Vorrichtung relativ zum Endotrachealtubus (110, 120, 200) festzulegen.

9. Eine Endotrachealtubus-Biegevorrichtung (400), umfassend:
einen ersten Zinken (412), der an einem ersten proximalen Ende an einer Basis befestigt ist und an einem ersten distalen Ende einen Vorsprung (415) aufweist; und
einen zweiten Zinken (410) und einen dritten Zinken (410), die jeweils an einem zweiten und dritten proximalen Ende an der Basis befestigt sind und über eine Brückenstruktur an einem zweiten und dritten distalen Ende miteinander verbunden sind, wobei eine Tasche (420) in der Brücke konfiguriert ist, um den Vorsprung (415) aufzunehmen, und wobei die Vorrichtung dazu ausgebildet ist, einen Endotrachealtubus (110, 120, 200, 540), der von dem ersten, zweiten und dritten Zinken (412, 410) aufgenommen wird, zu biegen, nachdem der Endotrachealtubus (110, 120, 200, 540) an einer ersten vorbestimmten Stelle in einer Trachea gesichert worden ist.

10. Die Endotrachealtubus-Biegevorrichtung nach Anspruch 9, wobei der erste, zweite und dritte Zinken (412, 410) konfiguriert sind, um den Endotrachealtubus (110, 120, 200, 540) um einen Winkel zu biegen, der dadurch entsteht, dass der Vorsprung (415) von der Tasche (420) erfasst wird.

11. Die Endotrachealtubus-Biegevorrichtung (500), umfassend:
einen Ringabschnitt (510);
einen Bandabschnitt (520), der vom Ringabschnitt ausgeht, wobei der Bandabschnitt dazu ausgebildet ist, einen Endotrachealtubus (110, 120, 200, 540) zu biegen, der in einer Ebene des Ringabschnitts über diesen verläuft, wodurch eine Biegung des Tubus um einen Winkel bewirkt wird, wobei der Endotrachealtubus (110, 120, 200, 540) an einer ersten vorbestimmten Stelle in einer Trachea befestigt ist, und wobei der Bandabschnitt Zähne (522) auf einer Oberfläche des Bandes beinhaltet; und
eine Sperrklinke (527), die in einer Öffnung im Ring gegenüber dem ausgehenden Abschnitt angeordnet ist und dazu ausgebildet ist, mit den Zähne (522) in Eingriff zu kommen, wodurch sich das Band festziehen lässt und ein Lösen des Bandes verhindert wird,
wobei der Ringabschnitt, der Bandabschnitt und die Sperrklinke (527) dazu ausgebildet sind, den Tubus um den Winkel zu biegen.

12. Die Vorrichtung nach irgendeinem der Ansprüche von 1 bis 11, wobei die Vorrichtung ein Acrylmaterial, ABS-Kunststoff, Polyethylen, Silikon, Harz oder ein anderes Kunststoff- oder Gummimaterial, Metall, eine Metalllegierung oder eine Kombination davon umfasst.

13. Die Endotrachealtubus-Biegevorrichtung nach irgendeinem der Ansprüche von 1 bis 12, wobei der Winkel zwischen 60 Grad und 90 Grad liegt.

14. Die Endotrachealtubus-Biegevorrichtung nach irgendeinem der Ansprüche von 1 bis 12, wobei der Winkel kleiner als 60 Grad oder größer als 90 Grad ist.

## Revendications

1. Un dispositif de courbure de tube endotrachéal (300), comprenant une portion dorsale (315) comprenant au moins une surface partiellement courbée créant un angle entre une extrémité proximale du dispositif et une extrémité distale du dispositif, sachant que le dispositif de courbure de tube endotrachéal comprend en outre :
une pluralité de portions d'extension courbées (320) configurées pour recevoir un tube endotrachéal (110, 120, 200) fixé à un premier emplacement prédéterminé dans la trachée contre la portion dorsale.

2. Le dispositif de courbure de tube endotrachéal d'après la revendication 1, sachant que chacune de la pluralité de portions d'extension courbées comprend une série de nervures structurées pour maintenir le tube endotrachéal (110, 120, 200) en place contre la portion dorsale.

3. Le dispositif de courbure de tube endotrachéal d'après la revendication 1 ou la revendication 2, sachant que la portion dorsale et la pluralité de portions d'extension courbées sont configurées pour courber le tube endotrachéal (110, 120, 200) selon ledit angle.

4. Le dispositif de courbure de tube endotrachéal d'après l'une quelconque des revendications de 1 à 3, comprenant en outre :
une structure de languette arrondie à proximité d'une première extrémité (112, 122) du dispositif, orientée longitudinalement le long du dispositif.

5. Le dispositif de courbure de tube endotrachéal d'après la revendication 4, sachant que la structure de languette arrondie présente une position de rotation s'étendant jusqu'à un bord de l'une parmi la pluralité de portions d'extension courbées lorsqu'on observe en coupe transversale au niveau de la première extrémité (112, 122) du dispositif, ou qu'elle présente une position de rotation opposée à l'une parmi la pluralité de portions d'extension courbées lorsqu'on observe en coupe transversale au niveau de la première extrémité (112, 122) du dispositif.

6. Le dispositif de courbure de tube endotrachéal d'après la revendication 4, sachant que la structure de languette arrondie présente une position de rotation située entre un bord de l'une parmi la pluralité de portions d'extension courbées et à l'opposé de ladite une parmi la pluralité de portions d'extension courbées (320) lorsqu'on observe en coupe transversale au niveau de la première extrémité (112, 122) du dispositif.

7. Le dispositif de courbure de tube endotrachéal d'après l'une quelconque des revendications de 4 à 6, sachant que la structure de languette arrondie présente un trou permettant le passage d'un fil de sécurité afin de permettre le retrait du dispositif depuis un patient.

8. Le dispositif de courbure de tube endotrachéal d'après l'une quelconque des revendications de 1 à 7, comprenant en outre :
une encoche allongée dans la portion dorsale, l'encoche allongée étant structurée pour s'engager avec un tube de ballonnet pilote (225) du tube endotrachéal (110, 120, 200), fixant ainsi une position du dispositif par rapport au tube endotrachéal (110, 120, 200).

9. Un dispositif de courbure de tube endotrachéal (400), comprenant :
une première branche (412) fixée à une base au niveau d'une première extrémité proximale et présentant un ergot (415) au niveau d'une première extrémité distale ; et
une deuxième branche (410) et une troisième branche (410) fixées chacune à la base aux extrémités proximales deuxième et troisième et reliées entre elles par une structure en pont aux extrémités distales deuxième et troisième, sachant que dans le pont une poche (420) est configurée pour capturer l'ergot (415), et sachant que le dispositif est structuré pour courber un tube endotrachéal (110, 120, 200, 540) reçu par les branches première, deuxième et troisième (412, 410) après que le tube endotrachéal (110, 120, 200, 540) a été fixé à un premier emplacement prédéterminé dans une trachée.

10. Le dispositif de courbure de tube endotrachéal d'après la revendication 9, sachant que les branches première, deuxième et troisième (412, 410) sont configurées pour courber le tube endotrachéal (110, 120, 200, 540) selon un angle dû au fait que l'ergot (415) est reçu par la poche (420).

11. Un dispositif de courbure de tube endotrachéal (500), comprenant :
une portion annulaire (510) ;
une portion en sangle (520) s'étendant à partir de la portion annulaire, sachant que la portion en sangle est structurée pour courber un tube endotrachéal (110, 120, 200, 540) passant à travers le plan de la portion annulaire dans un plan de la portion annulaire, provoquant une courbure du tube selon un angle, sachant que la tube endotrachéal (110, 120, 200, 540) est fixée à un premier emplacement prédéterminé dans la trachée, et sachant que la portion en sangle inclut des dents (522) sur une surface de la sangle ; et
un cliquet (527) situé dans un trou de l'anneau à l'opposé de la portion émergente, structuré pour s'engager avec les dents (522), permettant à la sangle de se resserrer et empêchant la sangle de se desserrer,
sachant que la portion annulaire, la portion en sangle et le cliquet (527) sont structurés pour courber le tube selon ledit angle.

12. Le dispositif d'après l'une quelconque des revendications de 1 à 11, sachant que le dispositif comprend un matériau acrylique, du plastique ABS, du polyéthylène, du silicone, de la résine ou un autre matériau plastique ou caoutchouteux, du métal, un alliage métallique ou une combinaison de ceux-ci.

13. Le dispositif de courbure de tube endotrachéal d'après l'une quelconque des revendications de 1 à 12, sachant que l'angle est compris entre 60 degrés et 90 degrés.

14. Le dispositif de courbure de tube endotrachéal d'après l'une quelconque des revendications de 1 à 12, sachant que l'angle est inférieur à 60 degrés ou supérieur à 90 degrés.
